# EUROPÄISCHE PATENTSCHRIFT

(11) **EP 1 473 561 B1**
(45) Veröffentlichungstag und Bekanntmachung des Hinweises auf die Patenterteilung: **14.06.2006**
(21) Anmeldenummer: 03405304.1
(22) Anmeldetag: 30.04.2003
(51) Int. Cl.: G01N 11/02, G01N 33/38

(54) **Verfahren und Vorrichtung zur Bestimmung der Konsistenz von wenig fliessfähigen Medien**
Method and device for determining the consistency of media with low flowability
Procédé et dispositif de détermination de la consistence des milieux à faible capacité d'écoulement

(43) Veröffentlichungstag der Anmeldung: 03.11.2004
(73) Patentinhaber: Hochschule Rapperswil, Institut für angewandte Umwelttechnik, 8640 Rapperswil (CH)
(72) Erfinder: Bunge, Rainer, Prof.Dr., 8057 Zürich (CH)

(56) Entgegenhaltungen:
- EP-A- 1 107 001
- DE-A- 4 315 167
- PATENT ABSTRACTS OF JAPAN vol. 2000, no. 22, 9. März 2001 (2001-03-09) & JP 2001 133380 A (KAYABA IND CO LTD), 18. Mai 2001 (2001-05-18)
- MURATA J ET AL: "New method of testing the flowability of grout" MAG CONCR RES;MAGAZINE OF CONCRETE RESEARCH DEC 1997 THOMAS TELFORD SERVICES LTD, LONDON, ENGL, Bd. 49, Nr. 181, Dezember 1997 (1997-12), Seiten 269-276, XP008024915
- WEIGAND J: "VISCOSITY MEASUREMENTS ON POWDERS WITH A NEW VISCOMETER" APPLIED RHEOLOGY, APPLIED RHEOLOGY, EDITORIAL OFFICE, ZURICH, CH, Bd. 9, Nr. 5, September 1999 (1999-09), Seiten 204-211, XP000930244

## Beschreibung

Die Erfindung fällt in das Gebiet der Konsistenzbestimmung. Sie betrifft ein Verfahren und eine Vorrichtung nach den Oberbegriffen der entsprechenden, unabhängigen Patentansprüche. Verfahren und Vorrichtung dienen zur Bestimmung der Konsistenz von wenig wenig fliessfähigen Medien, insbesondere von Frischbeton und Mörtel.

Frischbeton besteht im Wesentlichen aus Wasser, Zement, Kiessand und gegebenenfalls Betonzusatzmittel. Für die Eigenschaften von Beton ist insbesondere das Verhältnis der verwendeten Wassermenge und der verwendeten Zementmenge von grosser Bedeutung, der sogenannte "Wasser/Zement-Faktor" (W/Z). Durch Laborversuche kann ein "optimaler" W/Z ermittelt werden, z.B. durch die Bestimmung des Ausbreitmasses. Hierfür wird eine definierte Menge Frischbeton mit einer vorgeschriebenen Prozedur als Schüttkegel auf einer Platte abgelegt und der Kegel durch mehrere Erschütterungen der Platte ausgebreitet. Bei grossem W/Z ist der Durchmesser am Fuss des Betonkegels, also das Ausbreitmass, gross, bei kleinem W/Z klein. Im Mischwerk wird der so bestimmte optimale W/Z durch Einwägen der den Frischbeton bildenden Komponenten, also Wasser, Zement, Kiessand und Betonzusatzmittel eingestellt.

Problematisch ist hierbei, dass der zur Betonproduktion angelieferte Kies im Allgemeinen einen Feuchtigkeitsgehalt hat, der stark variieren kann und der nicht bekannt ist, so dass der Wassergehalt des Frischbetons daher nicht allein von der zudosierten Wassermenge abhängt. Aus diesem Grunde kann der tatsächliche W/Z von der Vorgabe abweichen, was zu Problemen führen kann.

Zur Lösung dieser branchenbekannten Probleme enthält die Patentliteratur zahlreiche Lösungsansätze. So wird in JP07280716A vorgeschlagen, die oben angesprochene, diskontinuierliche Bestimmung des Ausbreitmasses mit optischen Sensoren zu unterstützen. Zur kontinuierlichen Bestimmung des W/Z wird insbesondere vorgeschlagen, die

Leistungsaufnahme beim Rühren des Frischbetons zu messen (US5541855; GB2092308A). Nachteilig ist hierbei, dass sich beispielsweise die Korrelation zwischen dem W/Z und der Leistungsaufnahme am Rührer mit dessen fortschreitendem Verschleiss ändert. Andere Verfahren beruhen auf elektronischen Verfahren, z.B. Ultraschallmessungen (JP01010167A), die jedoch entweder nicht präzise, oder nicht robust genug für den Einsatz unter Betriebsbedingungen sind.

Aus der DE-A 43 15 167 ist ein Verfahren bekannt, bei dem die Rieselfähigkeit eines Schüttgutes anhand des Böschungswinkels eines Schtittkegels beurteilt wird. In einem Ausführungsbeispiel wird eine Vorrichtung vorgeschlagen, welche einen ersten Kanal mit einem grösseren Querschnitt , über welchen der Zufluss des Schüttgutes in ein Volumen zwischen dem ersten und einem zweiten Kanal erfolgt, und unterhalb dieses ersten Kanals einen zweiten Kanal mit einem kleineren Querschnitt, über welchen der Abfluss des Schüttgutes erfolgt. Der Gipfel des Schüttkegels reguliert den Zufluss ständig, so dass dank der Sperrwirkung des Schüttkegels das Volumen dem Abfluss entsprechend wieder aufgefüllt wird. Die Höhe des Schüttkegels bleibt in dieser Vorrichtung konstant, aber bei Änderung des Rieselverhaltens ändert der Böschungswinkel.

Die Erfindung stellt sich die Aufgabe, ein Verfahren und eine Vorrichtung zu schaffen, die es erlauben, die Konsistenz von wenig fliessfähigen (zähflüssigen bis krümeligen) Medien, insbesondere von Frischbeton, zu messen und zwar kontinuierlich, derart dass beispielsweise die Konsistenz von Frischbeton im Betonmischer direkt bestimmt und der optimale W/Z aufgrund dieser Bestimmung eingestellt werden kann. Verfahren und Vorrichtung gemäss Erfindung sollen eine befriedigende Genauigkeit liefern, sollen aber trotzdem einfach und robust sein, damit sie auch unter Betriebsbedingungen einsetzbar sind.

Diese Aufgabe wird gelöst durch das Verfahren und die Vorrichtung, wie sie in den Patentansprüchen definiert sind.

Nach dem erfindungsgemässen Verfahren wird die Konsistenz von wenig fliessfähigen Medien kontinuierlich bestimmt, indem an die konventionelle, diskontinuierliche Bestimmung des Ausbreitmasses eines statischen Schüttkegels anlehnend, ein quasistationärer Fliesskegel in einer geeigneten Weise bezüglich seiner Form oder Geometrie vermessen wird. Unter dem Begriff Fliesskegel wird eine im wesentlichen kegelförmig Anhäufung des wenig fliessfähigen Mediums verstanden, welcher Anhäufung kontinuierlich Medium zugeführt wird und von welcher Anhäufung kontinuierlich Medium abfliesst. Zur Bestimmung der Konsistenz wird beispielsweise die Höhe des Fliesskegels oder sein Volumen bestimmt, wobei das Volumen indirekt durch Bestimmung des Gewichtes bestimmt werden kann.

Die erfindungsgemässe Vorrichtung weist ein Fördermittel, eine Fliesskegelunterlage und Sensormittel auf. Das Fördermittel ist ausgerüstet für eine kontinuierliche Förderung des Mediums auf die Fliesskegelunterlage. Die Fliesskegelunterlage ist derart ausgebildet, dass das auf die Fliesskegelunterlage geförderte Medium von dieser wieder kontinuierlich abfliessen kann. Die Sensormittel sind zur direkten oder indirekten Bestimmung einer geometrischen Eigenschaft des sich durch kontinuierliche Förderung und kontinuierliches Abfliessen auf der Fliesskegelunterlage bildenden Fliesskegels ausgerüstet.

Es zeigt sich, dass solche Fliesskegel aus Frischbeton, die dadurch erzeugt werden, dass der Frischbeton durch ein senkrechtes Rohr auf einen um das obere Ende des Rohres angeordneten Kragen gefördert wird und vom Rand des Kragens wieder abfliesst, sehr stabile und reproduzierbare Formen, insbesondere eine stabile, reproduzierbare Höhe haben, die im Falle von Frischbeton sehr genau mit dem diskontinuierlich gemessenen Ausbreitmass von stationären Kegeln, also auch mit dem W/Z-Faktor korreliert ist. Mit entsprechenden Eichmessungen muss diese Korrelation für jede Art von Medium, dessen Konsistenz zu bestimmen ist, experimentell ermittelt werden.

Ferner zeigen Versuche überraschenderweise, dass die Höhe des quasi-stationären Fliesskegels nur sehr untergeordnet abhängt von der Fördergeschwindigkeit, mit der das Medium diesem zugeführt wird. Bei niedrigerer Fördergeschwindigkeit, beispielsweise aufgrund fortgeschrittenen Verschleisses der Fördereinrichtung, fliesst der Kegel entsprechend langsamer ab, ohne dass sich seine Höhe nennenswert verändert. Die Höhe des Fliesskegels verändert sich aber im Falle von Frischbeton ausserordentlich sensibel bei einer Veränderung des W/Z. Sie ist daher ideal als Messparameter für die Bestimmung des W/Z geeignet.

Das erfindungsgemässe Verfahren zeichnet sich trotz hoher Präzision vor allem durch Einfachheit und Robustheit aus. Die Vorrichtung ist überdies leicht zu reinigen. Das Verfahren eignet sich sowohl zum Einsatz auf einem industriellen Betonmischer, als auch für Laborversuche beispielsweise zur Bestimmung des optimalen W/Z unter kontrollierten Bedingungen.

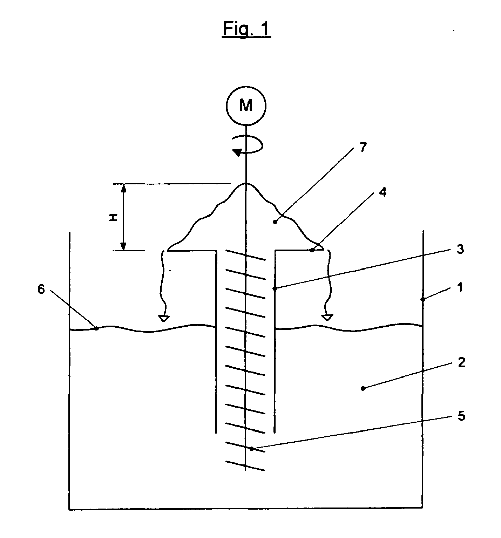

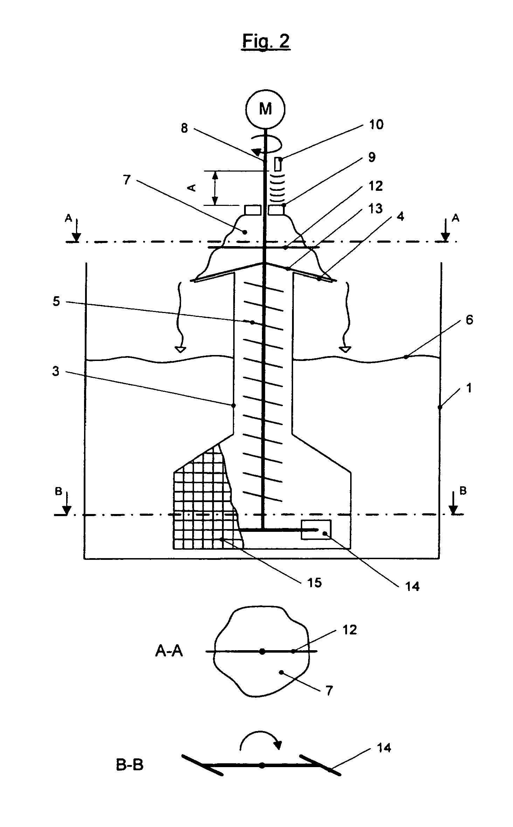

Das Verfahren gemäss Erfindung und zwei beispielhafte Ausführungsformen der entsprechenden Vorrichtung werden anhand der folgenden Figuren im Detail beschrieben. Dabei zeigen Figuren 1 und 2 eine erste und eine zweite beispielhafte Ausführungsform der erfindungsgemässen Vorrichtung.

Figur 1 zeigt eine erste beispielhafte Ausführungsform der erfindungsgemässen Vorrichtung. Diese Vorrichtung ist in einem mit dem Medium 2 gefüllten Behälter 1, beispielsweise in einem mit Frischbeton gefüllten Mischer, angeordnet und weist ein Förderrohr 3 mit einem an seinem oberen Ende angeordneten Rohrkragen 4 und eine im Förderrohr angeordnete Förderschnecke 5 zur Förderung des Mediums durch das Förderrohr 3 auf den Kragen 4 auf. Der Rohrkragen 4 ist also die Fliesskegelunterlage und das Förderrohr 3 zusammen mit der Förderschnecke 5 bilden das Fördermittel. Dieses wird von einem über dem Rohrkragen 4 angeordneten Motor (M) angetrieben. Die Vorrichtung ist derart im Behälter 1 montiert, dass sich das untere Ende des Förderrohres 3 unter der Mediumoberfläche 6 befindet und sein oberes Ende mit dem Rohrkragen 4 darüber.

Der Fliesskegel 7 bildet sich durch kontinuierliche Förderung des Mediums durch das Förderrohr 3 auf den Kragen 4. Das Medium fliesst über die Kragenkante ab, so dass sich an dieser Stelle der Fuss des Fliesskegels befindet.

Versuche zeigen, dass für eine in einem Betonmischer betriebene Vorrichtung gemäss Figur 1 der W/Z-Faktor des Betons in einem definierten Zusammenhang steht mit der Höhe (H) der Fliesskegelspitze über dem Rohrkragen 4. Bei besser fliessfähigen Mischungen (mit hohem W/Z) ist der Kegel niedrig, bei weniger fliessfähigen Mishungen (mit niedrigem W/Z) hingegen hoch.

Figur 2 zeigt eine zweite, beispielhafte Ausführungsform der erfindungsgemässen Vorrichtung. Auch diese dient beispielsweise zur Bestimmung der Konsistenz von Frischbeton und ist dafür in einem Betonmischer montiert. Die Vorrichtung weist dieselben Elemente auf wie die Ausführungsform der Figur 1, die auch mit denselben Bezugsziffern bezeichnet sind. Das Fördermittel ist auch hier eine Schneckenförderpumpe mit einem über dem Rohrkragen 4 angeordneten Motor M, einer Welle 8 und einer Förderschnecke 5, durch die der Frischbeton durch das Förderrohr 3 vertikal gefördert wird und aus der oberen Öffnung des Förderrohres 3 auf den Rohrkragen 4 ausfliesst. Über die Kante des Rohrkragens 4 läuft der Beton wieder zurück in den Mischer.

Zur Bestimmung der Höhe des sich auf dem Rohrkragen 4 ausbildenden quasi-stationären Fliesskegels 7 ist ein beispielsweise durch die Welle 8 vertikal zwangsgeführter Schwimmkörper 9 vorgesehen, wobei mit Hilfe beispielsweise einer stationären Ultraschall-Abstandmesssonde 10 der Abstand A zwischen dem Schwimmkörper 9 und der Sonde 10 und dadurch indirekt die Höhe H des Fliesskegels 7 über dem Rohrkragen 4 ermittelt wird. Das Signal der Sonde wird elektronisch verarbeitet und kann direkt zur Regulierung der Wasserzugabe in den Mischer verwendet werden.

Die Vorrichtung gemäss Figur 2 ist insbesondere ausgebildet für sehr wenig fliessfähige ("steife") hydraulisch abbindende Medien, deren W/Z sehr klein ist, so dass sie nicht mehr eigentlich flüssig sondern Krümelig sind. Beispiele für solche Medien sind Frischbeton und Mörtel. Der Rohrkragen 4 ist angeschrägt, um das kontinuierliche Abfliessen des Mediums zu erleichtern (Vermeidung von "toten" Zonen). Zur Durcharbeitung des Fliesskegels sitzen auf der Antriebswelle 8 im Bereich des Fliesskegels mit der Welle rotierende Elemente, beispielsweise eine Querstrebe 12 und ein Abstreifer 13, wobei die Querstrebe 12 sich im Fliesskegel 7 (siehe auch Schnitt A-A) und der Abstreifer 13 sich unmittelbar über dem Rohrkragen 4 bewegt. Querstrebe 12 und Abstreifer 13 bewegen das Medium im Fliesskegel 7 und fördern die Bildung eines symmetrischen Fliesskegels mit möglichst stabilen Abmessungen. Ein ähnlicher Effekt wird durch Vibration des Fliesskegels 7 erreicht. Am unteren Ende der Antriebswelle 8 sind ferner Schaufeln 14 montiert (siehe auch Schnitt B-B), durch die ein sehr wenig fliessfähiges Medium (z.B. wasserarmer, krümeliger Frischbeton) kontinuierlich in den Einzugsbereich des Förderrohres 3 gefördert werden kann. Der untere Teil der Vorrichtung kann auch mit einem Schutzkorb 15 umgeben sein, der die Verstopfung der Förderschnecke 5 durch grobe Kiespartikel verhindert.

## Patentansprüche

1. Verfahren zur Bestimmung der Konsistenz wenig fliessfähiger Medien, bei welchem das Medium auf einer Fliesskegelunterlage (4) zu einem Fliesskegel (7) aufgehäuft wird und kontinuierlich von der Fliesskegelunterlage (4) abfliesst und zur Bestimmung der Konsistenz des Mediums wenigstens eine geometrische Eigenschaft dieses Fliesskegels (7) bestimmt wird, **dadurch gekennzeichnet, dass** das Medium mit einem angetriebenen Fördermittel (3,5) kontinuierlich durch ein Förderrohr (3) auf einen an einem oberen Endes des Förderrohres angeordneter Rohrkragen (4) gefördert wird, welcher Rohrkragen die Fliesskegelunterlage (4) bildet, auf welcher das Medium **dadurch** kontinuierlich zu einem quasi-stationären Fliesskegel (7) aufgehäuft wird.

2. Verfahren nach Anspruch 1, **dadurch gekennzeichnet, dass** das Medium (2) ein hydraulisches Bindemittel enthält, insbesondere einen Zement.

3. Verfahren nach Anspruch 1 oder 2, **dadurch gekennzeichnet, dass** die geometrische Eigenschaft die Höhe (H) des Fliesskegels (7) ist.

4. Verfahren nach einem der Ansprüche 1 oder 2, **dadurch gekennzeichnet, dass** die geometrische Eigenschaft das Volumen des Fliesskegels (7) ist, und dass das Volumen indirekt durch Bestimmung des Gewichtes des Fliesskegels (7) bestimmt wird.

5. Verfahren nach einem der Ansprüche 1 bis 4 **dadurch gekennzeichnet, dass** der Fliesskegel (7) mechanisch durchgearbeitet oder vibriert wird.

6. Verfahren nach einem der Ansprüche 1 bis 5, **dadurch gekennzeichnet, dass** anhand der bestimmten geometrischen Eigenschaften des Fliesskegels (7) Steuerdaten erstellt werden, und dass mit Hilfe der Steuerdaten eine Zumischung von Flüssigkeit zum Medium (2) geregelt wird.

7. Vorrichtung zur Bestimmung der Konsistenz von wenig fliessfähigen Medien, mit einer Fliesskegelunterlage (4), welche Fliesskegelunterlage (4) derart ausgebildet ist, dass das Medium von dieser kontinuierlich abfliessen kann, und Sensormittel (9,10) zur Bestimmung einer geometrischen Eigenschaft eines sich auf der Fliesskegelunterlage bildenden Fliesskegels (7), **gekennzeichnet durch** ein angetriebenes Fördermittel (3,5) mit einem Förderrohr (3) zur kontinuierlichen Förderung des Mediums auf einen an einem oberen Ende des Rohres angeordneten Rohrkragen (4), welcher die Fliesskegelunterlage (4) bildet.

8. Vorrichtung nach Anspruch 8, **dadurch gekennzeichnet, dass** das Fördermittel ferner eine im Förderrohr (3) angeordnete Förderschnecke (5) aufweist, die über eine Welle (8) mit einem über dem Rohrkragen (4) angeordneten Antrieb (M) angetrieben wird.

9. Vorrichtung nach einem der Ansprüche 8 oder 9, **dadurch gekennzeichnet, dass** der Rohrkragen (4) von Innen nach Aussen schräg abwärts geneigt ist.

10. Vorrichtung nach einem der Ansprüche 9 oder 10, **dadurch gekennzeichnet, dass** die Sensormittel einen auf der Welle (8) vertikal beweglich geführten Schwimmkörper (9) und einen stationären, auf den Schwimmkörper (9) gerichteten Abstandssensor (10) aufweisen.

11. Vorrichtung nach einem der Ansprüche 7 bis 11, **dadurch gekennzeichnet, dass** den Fliesskegel (7) durcharbeitende Elemente vorgesehen sind.

## Claims

1. A method for determining the consistency of highly viscous media, with which the medium is accumulated on a flow cone underlay (4) into a flow cone (7) and continuously flows away from the flow cone underlay (4), and at least one geometric characteristic of this flow cone (7) is determined for determining the consistency of the medium, **characterised in that** the medium is continuously conveyed with a driven conveyor means (3, 5) through a conveyor tube (3) onto a tube collar (4) arranged at the end of the conveyor tube, said tube collar forming the flow cone underlay (4) on which the medium, by way of this, is continuously accumulated into a quasi stationary flow cone (7).

2. A method according to claim 1, **characterised in that** the medium (2) contains a hydraulic binding agent, in particular a cement.

3. A method according to claim 1 or 2, **characterised in that** the geometric characteristic is the height (H) of the flow cone (7).

4. A method according to one of the claims 1 or 2, **characterised in that** the geometric property is the volume of the cone (7), and that the volume is indirectly determined by way of determining the weight of the flow cone (7).

5. A method according to one of the claims 1 to 4, **characterised in that** the flow cone (7) is mechanically worked or vibrated.

6. A method according to one of the claims 1 to 5, **characterised in that** control data is set up on account of the determined, geometric characteristics of the flow cone (7), and that an admixture of fluid to the medium (2) is controlled with a closed loop with the help of the control data.

7. A device for determining the consistency of highly viscous media, with a flow cone underlay (4), said flow cone underlay (4) being designed in a manner such that the medium may continuously flow away from this, and sensor means (9, 10) for determining a geometric characteristic of a flow cone (7) forming on the flow cone underlay, **characterised by** a driven conveyor means (3, 5) with a conveyor tube (3) for the continuous conveying of the medium onto a tube collar (4) which is arranged at an upper end of the tube and which forms the flow cone underlay (4).

8. A device according to claim 7, **characterised in that** the conveyor means furthermore comprises a conveyor worm (5) arranged in the conveyor tube (3), said conveyor worm being driven with a drive (M) arranged above the tube collar (4), via a shaft (8).

9. A device according to claim 7 or 8, **characterised in that** the tube collar (4) is inclined obliquely downwards from the inside to the outside.

10. A device according to one of the claims 8 or 9, **characterised in that** the sensor means comprise a float (9) which is vertically movably guided on the shaft (8), and a stationary distance sensor (10) directed to the float (9).

11. A device according to one of the claims 8 to 10, **characterised in that** elements are provided which work the flow cone (7).

## Revendications

1. Procédé pour déterminer la consistance de milieux peu fluides pour lequel le milieu est accumulé en un cône d'écoulement (7) sur un support de cône d'écoulement (4) et s'écoule en continu du support de cône d'écoulement (4) et pour déterminer la consistance du milieu au moins une propriété géométrique de ce cône d'écoulement (7) est définie, **caractérisé en ce que** le milieu est transporté avec un moyen de transport entraîné (3, 5) en continu par un tube de transport (3) sur un collet de tube (4) placé à une extrémité supérieure du tube de transport, lequel collet de tube forme le support de cône d'écoulement (4) sur lequel le milieu est ainsi accumulé continuellement en un cône d'écoulement (7) quasi stationnaire.

2. Procédé selon la revendication 1, **caractérisé en ce que** le milieu (2) contient un liant hydraulique, en particulier un ciment.

3. Procédé selon la revendication 1 ou 2, **caractérisé en ce que** la propriété géométrique est la hauteur (H) du cône d'écoulement (7).

4. Procédé selon l'une des revendications 1 ou 2, **caractérisé en ce que** la propriété géométrique est le volume du cône d'écoulement (7) et que le volume est déterminé indirectement en définissant le poids du cône d'écoulement (7).

5. Procédé selon l'une des revendications 1 à 4, **caractérisé en ce que** le cône d'écoulement (7) est pétri ou vibré mécaniquement.

6. Procédé selon l'une des revendications 1 à 5, **caractérisé en ce que** des données de contrôle sont établies à l'aide des propriétés géométriques définies du cône d'écoulement (7) et qu'un mélange de liquide au milieu (2) est réglé à l'aide des données de contrôle.

7. Dispositif pour déterminer la consistance de milieux peu fluides avec un support de cône d'écoulement (4), lequel support de cône d'écoulement (4) est configuré de telle manière que le milieu peut s'écouler continuellement à partir de celui-ci et des moyens détecteurs (9, 10) pour déterminer une propriété géométrique d'un cône d'écoulement (7) qui se forme sur le support de cône d'écoulement, **caractérisé par** un moyen de transport entraîné (3, 5) avec un tube de transport (3) pour le transport continu du milieu sur un collet de tube (4) placé à une extrémité supérieure du tube, collet de tube qui forme le support de cône d'écoulement (4).

8. Dispositif selon la revendication 7, **caractérisé en ce que** le moyen de transport présente de plus une hélice transporteuse (5) placée dans le tube de transport (3) qui est entraînée par un arbre (8) avec un entraînement (M) placé au-dessus du collet de tube (4).

9. Dispositif selon l'une des revendications 7 ou 8, **caractérisé en ce que** le collet de tube (4) est incliné de l'intérieur vers l'extérieur en descendant en oblique.

10. Dispositif selon l'une des revendications 8 ou 9, **caractérisé en ce que** les moyens détecteurs présentent un corps flottant (9) guidé mobile verticalement sur l'arbre (8) et un détecteur de distance (10) stationnaire orienté vers le corps flottant (9).

11. Dispositif selon l'une des revendications 8 à 10, **caractérisé en ce qu'**il est prévu des éléments qui pétrissent le cône d'écoulement (7).
